# EUROPEAN PATENT SPECIFICATION

(11) **EP 2 508 134 B1**
(45) Date of publication and mention of the grant of the patent: **17.04.2019**
(21) Application number: 12004974.7
(22) Date of filing: 16.11.2006
(51) Int. Cl.: A61B 6/14, G03B 42/04

(54) **HOLDER DEVICE FOR DENTAL X-RAY DIGITAL SENSOR**
HALTEREINRICHTUNG FÜR EINEN DENTALEN RÖNTGENDIGITALSENSOR
DISPOSITIF SUPPORT POUR CAPTEUR NUMERIQUE DE RADIOLOGIE DENTAIRE

(30) Priority: 17.11.2005 US 737499 P
(43) Date of publication of application: 10.10.2012
(62) Divisional of application: 06837936.1
(73) Proprietor: DENTSPLY SIRONA Inc., York, PA 17401-2991 (US)
(72) Inventor: Steck, John, E., Round Lake, IL 60073 (US); Brown, Kimberly, C., Huntley, IL 60142 (US); Sullivan, Nicole, L., Elgin, IL 60120 (US)
(74) Representative: Hartz, Nikolai

(56) References cited:
- EP-A1- 1 623 673
- EP-B1- 0 397 599
- DE-U1- 20 314 972
- NL-C2- 1 026 122
- US-A- 5 652 779
- US-A- 5 677 537
- US-A1- 2004 096 040
- US-A1- 2005 013 412
- US-B1- 6 343 875

## Description

### TECHNICAL FIELD

A dental x-ray sensor holder is provided. The sensor holder has a geometry conducive to receive and secure a digital sensor of the type physically connected to another device such as a computer or the like via a connecting cable.
A generic dental x-ray sensor holder is known from NL 1 026 122.

### BACKGROUND OF THE INVENTION

Dental professionals have employed x-ray imaging for many years. A traditional dental x-ray procedure includes exposing an x-ray film to x-ray energy after it has passed through the target site. The film is developed and an image of the target site is achieved. It has also long been known that in order to obtain a useful image, the dental x-ray film must be positioned relative to the target site in a predetermined and secure manner. Many numbers of x-ray film holders and positioning devices have been developed, including for example, that shown in U.S. Pat. No. 3,473,026 .

More recently, many dental professionals have used digital x-ray sensors in place of traditional x-ray films. An example of such a sensor is shown for example in U.S. Pat. No. 6,652,141 .

As with x-ray films, it is necessary for the x-ray sensor to be secured in a predetermined position during the x-ray imaging procedure. In a manner similar to the use of x-ray films, holding and positioning devices have been developed for x-ray sensors.

A traditional problem with sensor holders is that connecting cable affixed to the sensor itself is cumbersome to position such that it does not interfere with the imaging procedure. Patient comfort is always a prime consideration in any dental procedure, and the positioning of the connecting cable is no different.

A need exists therefore, for a sensor holder than can be employed with digital sensor having a connecting cable. The holder should easily yet securely position and hold not only the sensor but also the connecting cable.

### SUMMARY OF THE INVENTION

According to the present invention, a holder for a digital dental x-ray sensor of the type having a connecting cable is provided, which has the features as defined by claim 1.

### BRIEF DESCRIPTION OF THE DRAWINGS

Fig. 1 is a perspective view of a sensor holder not forming part of the present invention, and configured for use particularly in anterior imaging procedures.
Fig. 2 is a perspective view of a sensor holder according to the concepts of the present invention, and configured for use particularly in bitewing imaging procedures,
Fig 3 is a perspective view of a sensor holder not forming part of the present invention, and configured for use particularly in posterior imaging procedures.
Fig. 4 is a perspective view of a sensor holder not forming part of the present invention, and configured for use particularly in endodontic imaging procedures.
Fig. 5 is a perspective view showing an exemplary step in removeably affixing a sensor having a connecting cable to a sensor holder not forming part of the present invention, and showing a support arm supporting both the inventive holder and a collimator ring for environmental purposes.
Fig. 6 is perspective view showing another exemplary step in removeably affixing a sensor to a holder as in Fig. 5 and being sequential to the step of Fig. 5.
Fig. 7 is perspective view showing another exemplary step in removeably affixing a sensor to a holder as in Fig. 5 and being sequential to the step of Fig. 6.
Fig. 8 is a perspective view of an embodiment of the invention.
Fig. 9 is a perspective view of another embodiment of the invention.
Fig. 10 is a perspective view of another embodiment of the invention.
Fig. 11 is a perspective view of a holding device not forming part of the present invention.
Fig. 12 is a perspective view of a holding device not forming part of the present invention.
Fig.13 is aperspective view of a holding device not forming part of the present invention.
Fig. 14 is a perspective view of a holding device not forming part of the present invention.

### PREFERRED EMBODIMENTS FOR CARRYING OUT THE INVENTION

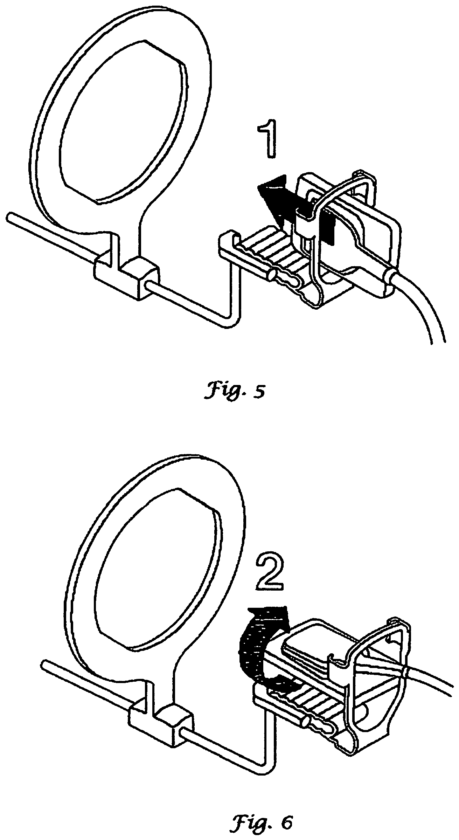

A sensor holder according to the present invention is shown by way of example on the drawing figures. While the present disclosure has application to any digital dental sensor, the holder of the claimed invention is adapted for use with sensors of the type having connecting cable. For environmental purposes, an exemplary sensor is shown on the drawings (Figs. 5-7).

Sensor is connected by an attached cable to a peripheral device (not shown) of some kind, such as a computer or the like. Although the present invention has application to any shape or size of sensors, the invention is exemplified herein with reference to a sensor having a generally rectangular configuration, such shape being standard in the industry.

As is conventional in the art, holder is preferably provided with a bite block of any configuration. According to a unique aspect, holder is provided with an upstanding frame, preferably affixed to or formed contiguously with bite block . By "upstanding" it is meant that frame is positioned at some angle to bite block. By nature, configuration and conventional function, bite block will be held by a clamping action caused by the patient biting upon the bite block itself. Hence, when in use, the bite block is at least somewhat parallel to the patient's occlusal plane (not shown). By being positioned at some angle with respect to the bite block and hence the occlusal plane, the upstanding frame is positioned to image either an upper or lower arch target site. An angle of about 90 degrees is often suitable although not necessarily a limitation of the present invention.

Frame preferably bounds or delimits a void area. An exemplary frame includes a base frame member which is positioned proximate to bite block . Two spaced and opposed side frame members extend from base frame member and may be joined by a distal frame member. Preferably although not necessarily, frame members lie in a similar plane.

At least one frame member carries a resilient latch finger. For example, side frame members are shown to carry opposed latch fingers respectively. By "resilient" it is meant that fingers can move slightly with respect to their respective frame members in a resilient manner. By suitably selecting the material of manufacture, such as a plastic material, fingers can be made to have such resiliency.

Further, fingers are each provided with means to accept a sensor in a snap-fit relation, and thereby to receive a sensor cooperatively therebetween. To facilitate such a snap-fit relation, an exemplary finger is shown as having a base portion and at least one curved portion contiguous therewith. It is preferred though not necessary that base portion and curved portion be integrally formed with the rest of holder . Similarly, finger has a base portion and a curved portion . By "curved portion" it is meant that base portions are positioned at some angle with respect to their respective curved portions . The angle can be sharp or curvilinear and can be any suitable angle. Preferably a pair of fingers are positioned in an opposing spaced relation as discussed above, such that curved portions
are inwardly directed toward each other. Because fingers are resilient, fingers can receive a sensor therebetween by slightly flexing and away from each other due to physical contact with the sensor. Once the sensor has traveled sufficiently between fingers, curved portions "close" upon sensor and hold sensor therebetween. Of course, any number of fingers such as fingers can be employed. For example, it is possible that only one finger is used wherein it is carried by distal frame member ; base frame member may also carry a finger similar to finger ; or all frame members may carry similar fingers (these embodiments not being shown). All such configurations are within the scope of the invention and are exemplified by the drawing figures.

As stated above, frame and its frame members such as frame members preferably bound or delimit a void area. Although the holder is preferred and exemplified with a complete frame around void, it is not necessary that frame completely surround void area (this configuration not being shown but which will be understood). The configuration of void and frame is such that when a sensor is so secured in place by said latch fingers, the connecting cable is positioned through said void area (Figs. 5-7). According to a method, a sensor with a connecting cable is inserted through frame void area from a side opposite the operational side of the holder when sensor is held therein for use. By" the operational side of the holder when sensor is held therein for use" it is meant that the sensor has a side which is positioned to receive x-ray energy during an imaging procedure and a side opposite side which normally carries an attachment point for cable. The side of sensor that receives x-ray energy in use is the operational side of sensor . Hence, " the operational side of the holder when sensor is held therein for use" is the same side when sensor is received and held in holder .

It will be appreciated that when sensor is inserted through void area, cable trails behind and through void area. At this point, sensor can be turned an rotated if needed, such that it properly aligns with frame and fingers (Figs. 6-7). It will also be appreciated that the steps of turning or rotating are not necessarily required. Once sensor is properly aligned it is physically received by and snap-fit into place by fingers as above described. It is to be appreciated that when sensor is so positioned and held by holder, cable is also secured and positioned by being held within void area. Thus the cable is out of the way or at least in a known position for the patient and the dental professional during an imaging procedure.

For environmental purposes, holder is shown in Figs. 5-7 as being affixed to a support arm and a collimator ring as it would be in actual use during au imaging procedure.

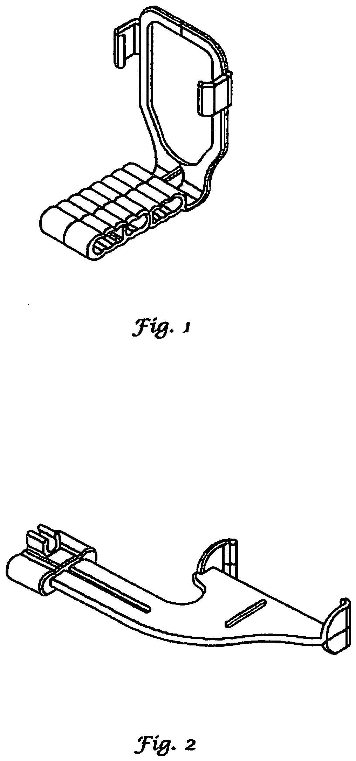

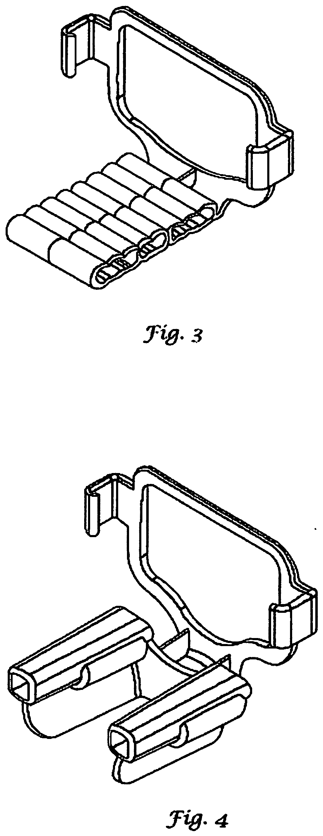

Figs. 1 and 3 show holders suitable for use in imaging procedures for anterior and posterior positions respectively. Fig. 4 shows a holder suitable for use in endodontic procedures wherein a bite block is formed by first and second spaced and opposing legs. The space between legs allows the placement and use of endodontic equipment such as files during the imaging procedure.

Fig. 2 shows a bitewing holder and which also has a cable conduit positioned thereon. It will be appreciated that according to the invention, conduit is useful to position and secure a cable during imaging procedures.

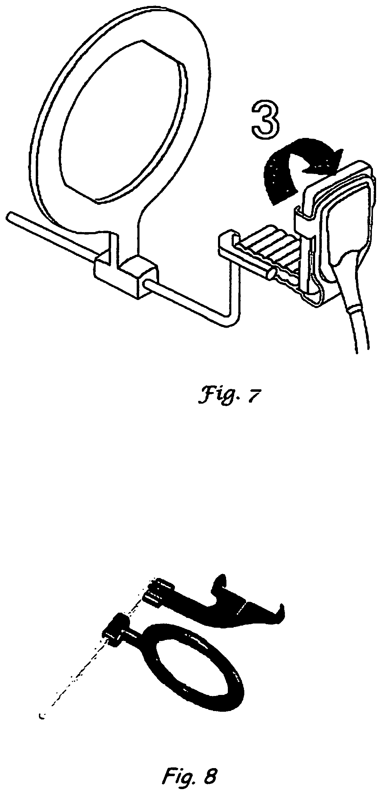

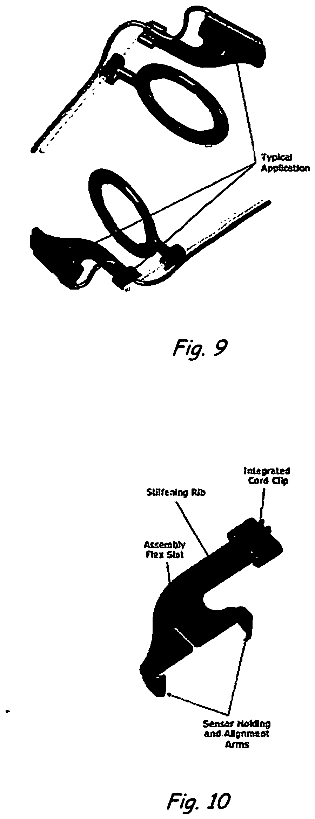

Figs. 8,9 and 10 show a digital sensor holding device according to the claimed invention. Said device designed to securely retain, centrally align, and perpendicularly align said sensor to an x-ray source. Sensor retention by means of tension pinch applied by integrated and opposing clamping arms of sufficient distance apart. Perpendicular alignment by means of this pinch in conjunction with making the contact surface of the clamping arms a sufficient length. Assembly of the sensor facilitated by means of a flex slot between said clamping arms which allows the unit to dynamically overcome the clamp arm tension pinch without putting excessive loading on said sensor. Central alignment of said sensor to the x-ray source facilitated by means of a stiffening rib to minimize deflection of the holding device relative to an attached alignment bar and ring. Cord retention of said sensor by means of an integrated cord clip. A typical use of this invention would be to facilitate the capture of optimally aligned digital dental radiography images.

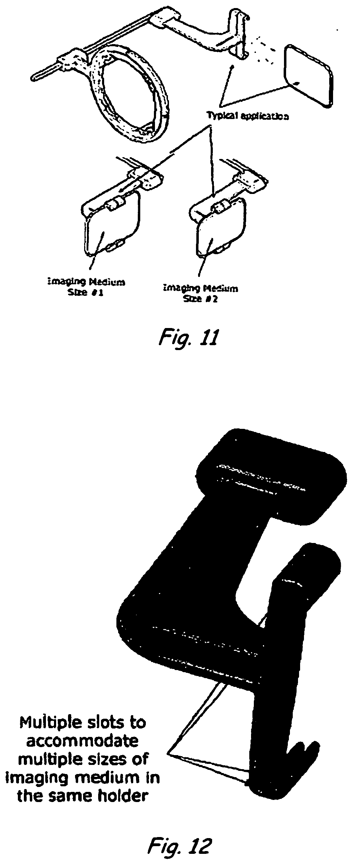

Figs. 11 and 12 show a radiographic imaging medium holding device. Said device designed to securely retain multiple sizes of x-ray film, phosphor plate, or similar medium in the same device. Medium retention by means of two or more pairs of slots of sufficient distance apart to capture by friction a variety of industry standard sizes of said medium. A typical use of this this holding device would be to facilitate the capture of optimally aligned digital dental radiography images.

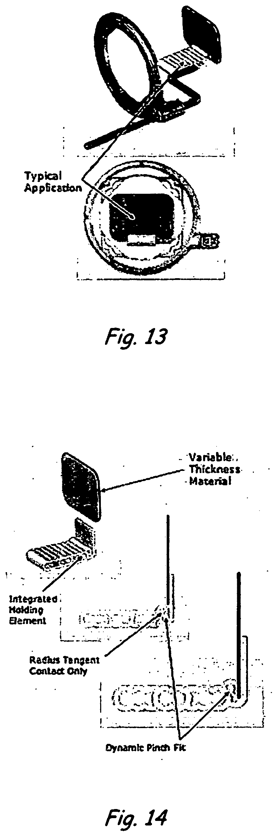

Figs. 13 and 14 show a holding device designed to accommodate thin materials of varying thicknesses by means of an integrated dynamic pinching element. Said pinching element configured in a flexible radial shape so as to isolate contact with the material to the tangent of the radius of the element. Said radial element configured to facilitate the inserting and removing of material and to minimize surface damage to the inserted or removed material. A typical use of this holding device would be to facilitate the use of x-ray film or x-ray phosphor plates in radiographical imaging. Another possible application would be the securing of developed x-ray film to an inspection light box.

It is evident therefore, that a sensor holder as shown and described carries out the intended purpose of the invention and otherwise provides a valuable contribution and advance to the art of sensor holders. The invention and its various embodiments have been exemplified herein by description and drawings without attempting to show all embodiments and variations .

## Claims

1. A holder for a digital dental x-ray sensor of the type having a connecting cable, the holder being designed to securely retain, centrally align, and perpendicularly align said sensor to an x-ray source, comprising:
(i) integrated and opposing clamping arms of sufficient distance apart so that perpendicular alignment by means of a tension pinch is provided in conjunction with a contact surface of the clamping arms which has a sufficient length so that sensor retention is provided,
(ii) a flex slot between said clamping arms allowing the unit to dynamically overcome the clamp arm tension pinch without putting excessive loading on said sensor assembly of the sensor,
(iii) a stiffening rib to minimize deflection of the holder relative to an alignment bar and collimator ring when such alignment bar having a collimator ring is attached to the holder for facilitating central alignment of said sensor to an x-ray source, **characterized in that**
- a cord clip is integrated with the holder for providing cord retention; and **in that**
- the holder is designed for being affixed to an alignment bar having a collimator ring during an imaging procedure.

## Patentansprüche

1. Halter für einen dentalen Röntgendigitalsensor des Typs, der ein Kabel aufweist, wobei der Halter so gestaltet ist, dass er den Sensor sicher an einer Röntgenquelle hält, zentral ausrichtet und rechtwinklig ausrichtet, umfassend:
(i) integrierte und gegenüberliegende Klemmarme, die einen ausreichenden Abstand voneinander entfernt sind, sodass die rechtwinklige Ausrichtung mittels einer Spannbacke zusammen mit einer Kontaktfläche der Klemmarme bereitgestellt wird, die eine ausreichende Länge hat, um für Sensorhalt zu sorgen,
(ii) einen flexiblen Schlitz zwischen den Klemmarmen, der es der Einheit ermöglicht, die Klemmarm-Spannbacke dynamisch zu überwinden, ohne übermäßige Last auf die Sensorbaugruppe des Sensors aufzuwenden,
(iii) eine Versteifungsrippe, um die Durchbiegung des Halters in Bezug auf eine Ausrichtungsstange zu minimieren, und einen Kollimatorring, wenn eine solche Ausrichtungsstange, die einen Kollimatorring aufweist, am Halter angebracht ist, um die zentrale Ausrichtung des Sensors an einer Röntgenquelle zu erleichtern, **dadurch gekennzeichnet, dass**
- eine Kabelklemme in den Halter integriert ist, um für Kabelhalt zu sorgen; und dadurch, dass
- der Halter für das Anbringen an einer Ausrichtungsstange, die einen Kollimatorring aufweist, während eines Bildgebungsverfahrens gestaltet ist.

## Revendications

1. Support pour un capteur numérique de radiologie dentaire du type ayant un câble de connexion, le support étant conçu pour retenir solidement, aligner au centre et aligner perpendiculairement ledit capteur sur une source de rayons X, comprenant :
(i) des bras de serrage intégrés et opposés espacés d'une distance suffisante pour qu'un alignement perpendiculaire au moyen d'un pincement de tension soit assuré conjointement à une surface de contact des bras de serrage ayant une longueur suffisante pour assurer la retenue du capteur,
(ii) une fente de souplesse entre lesdits bras de serrage permettant à l'unité de surmonter dynamiquement le pincement de tension des bras de serrage sans appliquer une charge excessive audit assemblage de capteur du capteur,
(iii) une nervure de raidissement pour minimiser une déviation du support par rapport à une barre d'alignement et un anneau collimateur lorsque cette barre d'alignement ayant un anneau collimateur est attachée au support pour faciliter un alignement central dudit capteur avec une source de rayons X, **caractérisé en ce que**
- une pince de cordon est intégrée au support pour assurer une retenue de cordon ; et **en ce que**
- le support est conçu pour être fixé à une barre d'alignement ayant un anneau collimateur pendant une procédure d'imagerie.
